# EUROPEAN PATENT SPECIFICATION

(11) **EP 2 988 606 B1**
(45) Date of publication and mention of the grant of the patent: **14.06.2017**
(21) Application number: 14724330.7
(22) Date of filing: 23.04.2014
(51) Int. Cl.: A23G 4/20, A61K 8/37, A61K 8/42, A61K 8/49, A61K 8/81, A61K 8/02, A61K 8/64

(54) **CHEWING GUM WITH LONG-LASTING FRESHNESS AND ITS MANUFACTURING PROCESS**
KAUGUMMI MIT LANGANHALTENDER FRISCHE UND DESSEN HERSTELLUNGSVERFAHREN
GOMME A MACHER A FRAICHEUR PROLONGEE ET SON PROCEDE DE PREPARATION

(30) Priority: 24.04.2013 IT MI20130685
(43) Date of publication of application: 02.03.2016
(73) Proprietor: Perfetti Van Melle S.p.A., 20020 Lainate (Milano) (IT)
(72) Inventor: COLLE, Roberto, I-20020 Lainate (MI) (IT); BALDI, Gianni, I-20020 Lainate (MI) (IT); DELEO, Maurizio, I-20020 Lainate (MI) (IT)
(74) Representative: Bianchetti Bracco Minoja S.r.l.
(86) International application number: PCT/EP2014/058179
(87) International publication number: WO 2014/173922

(56) References cited:
- WO-A1-90/11020
- WO-A1-2005/082154
- WO-A1-2011/159935
- WO-A2-2011/158123
- AU-B2- 2002 318 886
- US-A- 4 740 376
- US-A1- 2006 263 475
- US-A1- 2008 050 605

## Description

The present invention relates to chewing gums that impart a long-lasting cooling sensation during chewing.

### Prior art

One of the main reasons for consumption of chewing gum is the desire for a cooling sensation. This can be provided naturally by menthol which, however, has a strong mint flavour and a bitter taste. For this reason, specific flavours, called cooling agents or coolants, which belong to the families specified below, have been developed in order to increase the intensity of the cooling sensation.

### Mixtures of carboxamides

It has been found that some carboxamides increase the cooling sensation, for example when mixed with menthol in various percentages (US 5009893 and US 5698181), or in flavouring compositions containing fruit flavours, as reported inEP 1 003 383 B2.

Mixtures of carboxamides with menthyl acetate in spray-dried form (US6497859) and mixtures with menthol and/or menthyl esters are also known (WO 2010-096542, US 2007-0221236, US 2007-077331).

The overall teaching is to use mixtures with a precise balance between carboxamides and other ingredients of the cooling mixture, in order to maximise the cooling effect and minimize undesirable aspects such as bitter flavours and burning sensations.

EP 1935252 describes cooling agents in chewing gums coated with xylitol, wherein the latter is reduced as a result of using the cooling agents. The cooling agents can be mixed together, and the mixtures optionally encapsulated. Encapsulation methods and materials are cited generically.

### Monomenthyl succinate and other menthyl esters

US 5725865 and US 5843466 disclose compositions containing a specific menthyl ester: monomenthyl succinate (CAS77341-67-4). The listed compositions include chewing gum. Monomenthyl succinate can be mixed with solvents, in particular polar solvents such as alcohols and propylene glycol, with flavours or other cooling agents. In view of the wide range of fields of application, the doses are necessarily generic (0.001-1%).

### Cooling flavours in the form of particulate solid matter

Cooling flavours in the form of particulate solid matter are disclosed in WO 2011/159935 and EP 2124601, both of which relate to mixtures of different types of cooling agents encapsulated together.

WO 2011/159935 discloses mixtures of many different cooling agents. For example, menthol is mixed with menthyl succinate, isopulegol and menthane diol. The mixtures can, in turn, be encapsulated in a plurality of materials and methods.

EP 2124601 illustrates filled and coated chewing gums, wherein the filling consists of particulate matter. Said particulate matter consists of granules with dimensions of 980-1900 µm, comprising xylitol together with menthol flavours and cooling agents such as menthyl succinate, lactate or carboxamides. The granules provide a rapid release of freshness and a crunchy sensation.

The general teaching is therefore to mix various types of cooling agents, including those belonging to different classes of compounds (such as esters and amides), to achieve the desired effect.

### Solid particulate matter containing aspartame

Compositions in the form of solid particulate matter for the slow release of sweeteners, in particular aspartame, are known. These are obtained by blending aspartame with a melted polymer and a plasticiser at hot temperatures. The mixture is left to cool and reduced to the desired particle size at a later stage. This process is used, for example, in EP 229000, US 4740376 and EP 273009, which illustrate compositions for the slow release of sweeteners in polyvinyl acetate of various grades. Encapsulation of flavours, including essential oils, is also generically cited.

WO 2006/127065 and WO 2006/127074 generically illustrate many encapsulation possibilities and combinations. Sweeteners, acids, flavouring compounds, agents with pharmacological activity, vitamins, minerals, breath-freshening agents, tooth-whitening agents, cleansing agents, cooling or heating agents, throat-soothing substances, spices, caffeine, medicaments, etc. are cited as active agents which can be encapsulated.

Although different types of cooling agents are known, and although controlled-release systems of intensive sweeteners and flavours are known, it is still necessary to find formulations that guarantee a long-lasting cooling sensation, and are simple and cheap to make.

### Description of the invention

It has now been found that a chewing gum gives the consumer a cooling sensation when said gum comprises a region characterised by the presence of gum base, at least one sweetener, and a cooling synergistic combination of two ingredients a) and b) which are separated from one another:
a) a cooling flavour in the form of solid particulate matter comprising a vinyl polymer, a dipeptide sweetener and at least one N-substituted p-menthane-carboxamide of formula I 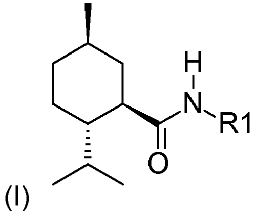 wherein R1 is C1-C10 alkyl, C1-C10 alkoxycarbonylmethyl, heteroaryl-C1-C5 alkyl or phenyl, optionally substituted by C1-C5 alkoxy and/or cyano groups.
b) a cooling mixture comprising one or more organic (C3-C10) mono- and di-acids esterified with one or two menthol groups and optionally at least one lipophilic ingredient, said mixture being evenly dispersed in said region.

The Applicant has found that chewing gum containing the synergistic combination provides a more intense and long lasting cooling sensation than a chewing gum with its constituents when administered to a consumer, even after 20 minutes or more of chewing, for example after 30, 40 or 50 minutes chewing.

Menthol esters have formula II 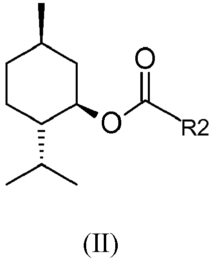 wherein R2 is a C2-C10 alkyl substituent optionally substituted by one or more carboxyl groups which, in turn, can be esterified with menthol.

The Applicant has also found that, contrary to the teachings of the prior art, the cooling sensation released by the product and perceived by a consumer lasts much longer than that obtainable with known manufacturing techniques, if, the cooling agents belonging to different classes of compounds, such as esters and amides, are separated. A further advantage on the same lines is obtained by including p-menthane-carboxamides in solid formulations with dipeptide sweeteners, there being no need to mix them with menthol as taught by the prior art. Menthol esters can be advantageously used in liquid mixtures with lipophilic ingredients.

The chewing gums of the invention can consist of a single region comprising at least one gum base and a sweetener (sticks or slabs) or can comprise a number of regions, one of which comprises at least one gum base and a sweetener (chewing gum dragee or pellets, filled dragee, filled slabs and multilayer gums).

Another object of the invention is a process for the preparation of chewing gums which involves preparing a mixture by adding the following ingredients in a mixer and mixing them for a total time (tₜₒₜ):
I. gum base;
II. at least one sweetener;
III. a cooling flavour in the form of solid particulate matter comprising a vinyl polymer, a dipeptide sweetener and an N-substituted p-menthane-carboxamide of formula I;
IV. a liquid cooling premix comprising one or more organic C3-C10 mono- and di-acids esterified with one or two menthol groups, and optionally at least one lipophilic ingredient, said premix being evenly dispersed in said region.

Ingredients III) and IV) are added at two different stages of mixing: t_{III}<tₜₒₜ and t_{IV}<tₜₒₜ. The absolute difference between t_{III} and t_{IV} is preferably greater than 30 seconds, more preferably greater than 60 seconds, and even more preferably greater than 120 seconds.

The mixer can be a double-Z mixer or a continuous extruder. During the mixing stage it is preferable to control the temperature of the mixture so that it is less than 100°C, preferably less than 70°C, and even more preferably less than 55°C.

"Liquid cooling premix" means a preparation of a mixture in liquid form which is obtained before mixing the ingredients in a mixer. The premix remains in liquid form until mixing. In the finished product, the premix is mixed with, dispersed through, adsorbed on the surface of and/or absorbed into the other ingredients of the mixture, and is described as the "cooling mixture".

The liquid cooling premix is prepared, preferably at a temperature of less than 50°C, before addition in the mixer or continuous extruder.

Adding at different times, during mixing, the cooling flavour in the form of solid particulate matter and the liquid cooling premix enables the former to maintain its properties and the latter to interact freely with the other ingredients of the mixture. Addition time t_{IV}, of the liquid cooling premix, is preferably less than addition time t_{III}, of the cooling flavour in the form of solid particulate matter, and simultaneously the difference t_{III}-t_{IV}>30s, preferably t_{III}-t_{IV}>60s; t_{III}-t_{IV}>120s. In this way, carboxamides and menthol esters are also kept separate during the process of obtaining the chewing gum according to the invention, as well as in the finished product.

When the mixture has been obtained, it is reduced to a plurality of chewing gum pieces, such as sticks or slabs, or a plurality of cores, by one or more of the following processes: extrusion, die forming, cutting, rolling and scoring.

A substance having the function of filling of the chewing gum pieces or cores can also be introduced into the mixture. The filling can be liquid, solid, granular or gelatinous, and is introduced after or at the same time as the extrusion stage and before the moulding, cutting or rolling stage. The insertion of a filling can contribute to the cooling sensation imparted by the invention by the use of further flavours, cooling agents, or the use of solids or particulates with negative heat of solution, including many polyalcohols.

The invention can be completed by further application of one or more layers of hard or soft coating and combinations thereof. Coating can be performed in pans. Coating can comprise the application of powders, application of one or more layers of smoothening syrup and application of one or more layers of gloss. As an alternative to gloss, a rough finishing can be obtained and, in a particular embodiment, a satin finishing.

In the context of the invention, "satin effect" means a low level of roughness, characterised by an average roughness Ra of between 1 and 6 µm and/or a roughness Rz between 5 and 30 µm.

Flavours, cooling agents and mixtures, together with ingredients with negative heat of solution that reinforce the cooling sensation imparted by the invention, can also be used in the coating region.

### Description of Figure

Figure: Chart showing the intensity of the cooling sensation on a scale of 0 to 100 for a series of chewing gums manufactured according to the prior art and two chewing gums of the invention, chewed for up to 60 minutes.

### Detailed description of the invention

In the context of the present invention, "cooling sensation" means a stimulus transmitted by the nerve endings present in the oral cavity and nose to the central nervous system that convey said sensation, which is not necessarily connected with a reduction in temperature. Compounds were recently synthesised which impart said sensation in a more intense or lasting way than menthol, without the association of a specific flavour. Said compounds are conventionally known as (and hereinafter called) "cooling agents" or "coolants". They can belong to different classes of compounds and preferentially act in particular areas (e.g. the palate, tongue and throat). Although they do not impart a particular flavour they are included in lists of flavouring substances; the preparations containing them are therefore called "flavours" or "cooling flavours".

The chewing gum of the invention comprises a region characterised by the presence of gum base, at least one sweetener, and a synergistic cooling combination of two ingredients which are separated from one another, the first in the form of solid particulate matter, and the second in the form of a mixture evenly dispersed in said region.

### Cooling flavour in the form of solid particulate matter

"Particulate matter" means a set of more or less rigid granules, characterised by a certain particle-size distribution. In the particulate matter, over 40% by weight of the particles are typically smaller than 1000 µm and larger than 250 µm. In one embodiment, more than 80% by weight of the particles are smaller than 1000 µm and larger than 250 µm. Intermediate forms of embodiment are possible, including those wherein the average particle size is between 400 and 600 µm or between 600 and 800 µm. The single particles can appear under the microscope in different shapes, including spheroids and flakes with sharp edges.

The solid particulate matter of the present invention can be obtained by known methods. However, contrary to the teachings of the prior art, it has been found advantageous to include in the particulate matter at least one N-substituted p-menthane-carboxamide of formula I, a dipeptide sweetener and polyvinyl acetate.

N-substituted p-menthane carboxamides are represented by formula I 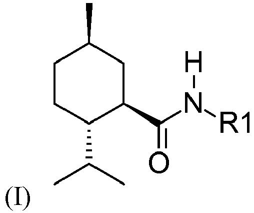 wherein R1 is C1-C10 alkyl, C1-C10 alkoxycarbonylmethyl, heteroaryl-C1-C5 alkyl or phenyl, optionally substituted by C1-C5 alkoxy and/or cyano groups.

The N-substituted p-menthane-carboxamide is preferably selected from N-ethyl-p-menthane carboxamide (WS3), N-ethoxycarbonylmethyl-p-menthane carboxamide (WS5), N-p-methoxyphenyl-p-menthane carboxamide (WS12), N-p-benzeneacetonitrile-menthanecarboxamide, N-(2-(pyridin-2-yl)ethyl)-3-p-menthanecarboxamide and combinations thereof, corresponding to the formulas: 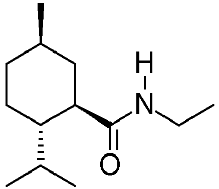 N-ethyl-p-menthane carboxamide (WS3), CAS 39711-79 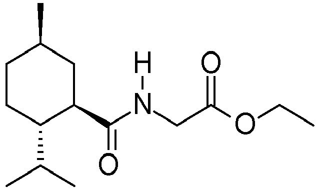 N-ethoxycarbonylmethyl-p-menthane carboxamide (WS5), CAS 68489-14-5 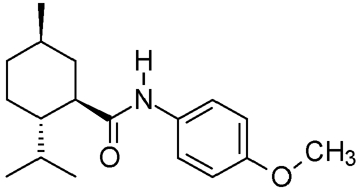 N-p-methoxyphenyl-p-menthane carboxamide (WS12), CAS 57233-03-1 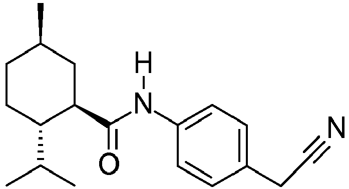 N-p-benzeneacetonitrile menthane-carboxamide, CAS: 852379-28-3 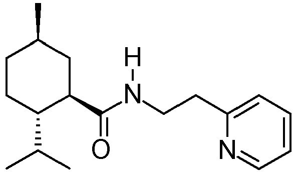 N-(2-(pyridin-2-yl)ethyl)-3-p-menthane carboxamide, CAS 847565-09-7.

The dipeptide sweetener is an intensive sweetener, i.e. it imparts sweetness to a greater extent than sugar. Dipeptide sweeteners are characterised by a backbone resulting from two amino acids bonded by an amido bond. The amino acids can present modifications from natural amino acids. Dipeptide sweeteners are preferably selected from aspartame, neotame, advantame and alitame and combinations thereof.

The vinyl polymer is preferably selected from polyvinyl acetate, polyvinyl acetate/polyvinyl laurate copolymer, polyvinyl acetate/polyvinyl versatate/polyvinyl alcohol terpolymer, polyvinylpyrrolidone, crosslinked polyvinylpyrrolidone, polyvinyl alcohol and mixtures thereof. Particularly preferred are polyvinyl acetate and polyvinyl acetate/polyvinyl versatate/polyvinyl alcohol terpolymer, alone or mixed together.

The vinyl acetate polymer, included in the ingredients of the matrix of solid particulate matter of the invention, retains the cooling agent and the sweetener, and releases them gradually during chewing.

Polyvinyl acetate is particularly preferred. Many different grades of polyvinyl acetate are available on the market, some of which, of food grade, are used in the manufacture of gum base. The use of dipeptide sweeteners, in a matrix of vinyl polymers, and in particular of polyvinyl acetate, makes the freshness imparted by the cooling agents deriving from p-menthane-carboxamide more perceptible, and also influences the rheological properties of the vinyl acetate polymer, producing a solid particulate which is neither excessively fragile nor excessively soft, and characterised by slow release of the cooling agent. The molecular weight of polyvinyl acetate can vary within wide limits, from 5,000 to 20,000 UMA, preferably from 15,000 to 30,000. Alternatively, a grade between 35000 and 100000 UMA can be used. Polyvinyl acetates of different grades can be mixed. Polyvinyl alcohol, generated in the industrial finishing process, may be present in polyvinyl acetate.

Other polymers, customarily used in gum base formulation, can be used in combination with the vinyl acetate polymer.

Additives, plasticisers, colorants, polyalcohols, acids and other flavouring substances can be present in the granulate. In particular, it is preferable to use plasticisers in quantities lower than 20% by weight of the granulate. Suitable plasticisers are mono-, di- or triglycerides, lecithins, mono-, di- or triesters of citric acid, sebacic acid esters, sucrose esters and mixtures thereof. Mono-, di- and triglycerides comprise natural, fractionated, partly or totally hydrogenated oils and fats.

In one embodiment, the cooling flavour in the form of particulate matter does not contain solvents.

In a particular form of embodiment, the cooling flavour in the form of particulate matter does not contain menthol esters.

Some ratios, between the main ingredients of the cooling flavour in the form of particulate matter, are preferred because they facilitate better perception of the freshness in the chewing gum. In particular, a ratio between N-substituted p-menthane-carboxamide : dipeptide sweetener : polyvinyl acetate ranging from a minimum of 0.02:1:0.75 to a maximum of 8:1:9 is preferred.

The three main ingredients can be present in the particulate matter in the following percentages by weight: N-substituted p-menthane-carboxamide 1% to 10%, dipeptide sweetener 5% to 50%, and polyvinyl acetate 30% to 90%.

Finally, it is preferable for the cooling flavour in the form of solid particulate matter to be contained in the chewing gum in a percentage ranging from 0.1 % to 10% by weight of the chewing gum, and even more preferably from 1% to 5%.

### Cooling mixture with menthol esters

The second crucial ingredient of the invention is a cooling mixture, whose ingredients are mixed before the chewing gum is made. The manufacture of the chewing gum also requires mixing the ingredients such as at least one gum base, at least one sweetener, a cooling flavour in the form of solid particulate matter, and a cooling mixture.

The cooling mixture comprises one or more organic C3-C10 mono- and di-acids esterified with one or two menthol groups, and optionally at least one lipophilic ingredient.

"Lipophilic ingredient" means an ingredient of the mixture which is substantially insoluble or immiscible in water.

Lipophilic ingredients which can be used within the scope of the present invention are preferably oils, animal fats, vegetable fats, essential oils, and flavouring solvents.

Before being added to the other ingredients of the invention, the mixture is in liquid form. The preferred menthol esters are monomenthyl glutarate, dimenthyl glutarate and monomenthyl succinate.

Said cooling mixture in liquid form preferably comprises, as a percentage of the mixture, 1% to 70% monomenthyl glutarate, 1% to 70% dimenthyl glutarate and 0.5% to 50% monomenthyl succinate.

The liquid cooling mixture preferably consists of monomenthyl glutarate, dimenthyl glutarate, monomenthyl succinate and triglycerides.

The cooling mixture can advantageously include, as lipophilic ingredient, C₁-C₃ chain triglycerides ranging from 1% to 70% by weight of the mixture and C₆-C₁₂ chain triglycerides ranging from 1% to 70% by weight of the mixture.

In one embodiment, the cooling mixture in liquid form ranges from 0.2% to 5% of the chewing gum.

The cooling and lipophilic ingredients are mixed together before preparation of the region comprising gum base and at least one sweetener, to form a liquid cooling premix. Said premix is prepared with rotor mixers, anchor mixers, planetary mixers or manual mixing. It can be stored at room temperature until addition at the mixing stage. The liquid mixture is then dispersed evenly in the region of the chewing gum characterised by the presence of gum base and at least one sweetener.

The inventive gum characterised by the ingredients described imparts a cooling sensation when administered to a consumer, even after 10 minutes' chewing. In particular, it imparts a cooling sensation even after or at 20, 30, 40 or 50 minutes' chewing.

### Additional cooling agents

The chewing gum can also contain a further cooling flavour in spray-dried, liquid or encapsulated form, wherein the flavouring substances comprise one or more menthane carboxamides and menthol esters. In particular, the use of N-p-benzeneacetonitrile-menthane carboxamide (CAS 852379-28-3), N-(2-(pyridin-2-yl)ethyl)-3-p-menthane carboxamide (CAS 847565-09-7) and menthol esters, alone or mixed together, is preferred.

A flavouring in spray-dried form on gum arabic, containing a mixture of menthyl lactate with N-(2-(pyridin-2-yl)ethyl)-3-p-menthane carboxamide substantially devoid of menthol, is preferably used. In particular, the flavouring substances can be present in the following percentages of the total flavouring substances: menthyl lactate over 50%, N-(2-(pyridin-2-yl)ethyl)-3-p-menthane carboxamide over 3%, menthol under 1%. The flavouring substances are preferably present in the following percentages of the total flavouring substances: menthyl lactate between 30% and 60%, N-(2-(pyridin-2-yl)ethyl)-3-p-menthane carboxamide between 20 and 40%, menthol absent. The addition of said additional mixture of cooling agents in spray-dried form increases freshness during chewing in the medium-long term.

In chewing gum containing several regions, the additional cooling agents can be present in the region characterised by the presence of gum base and/or in the other regions.

### Additional flavours

The inventive chewing gum can also contain flavours such as flavouring preparations (including essential oils), natural and artificial flavouring substances, flavours deriving from heat treatments, smoked flavouring substances, solvents, flavour's additives, supports, modifiers and mixtures thereof such as known by those expert in the field.

Said flavours are typically fat-soluble, and liquid at room temperature. However, solid forms of these flavours, supported on one or more carriers, are readily available on the market.

Release systems obtained as disclosed in EP 2560501 can also be used.

### Chewing gum

Chewing gum can be made in any shape, such as sticks, slabs, cigarettes, and cushion, ball, cube and disc shapes. The gum can contain a region consisting of a filling, which may be totally included in the chewing gum region or be visible from the exterior on one or more sides of the chewing gum. Said filling can be liquid, solid, granular or gelatinous.

Said chewing gum can be also be coated, namely a chewing gum containing at least one central region, comprising the gum base and a sweetener, called the core, and an outer region, called the coating.

### Region with gum base and sweetener

The first stage of the chewing gum preparation process involves preparing the region with gum base and at least one sweetener. The crucial step is to obtain a gum dough. Final pieces of chewing gum or centers of dragees are obtained from the gum dough.

The gum base used for the present invention is manufactured with known methods and ingredients.

The sweetener can be selected from sugars (in solid form, such as sucrose and glucose, or in the form of syrup, such as glucose syrup), polyols (in solid form, such as sorbitol, or in the form of syrup, such as maltitol syrup), and combinations thereof. The polyols can be selected from sorbitol, mannitol, maltitol, isomalt (a mixture of glucopyranosyl mannitol and glucopyranosyl sorbitol), erythritol, xylitol, maltitol syrup and mixtures thereof. Various particle size grades are available for each of the ingredients listed, which can be used mixed together, to modulate the hardness, flavour and tactile sensation of the chewing gum composition.

The gum dough may also contain one or more of the following substances: intensive sweeteners, flavouring agents in solid or liquid form, wetting agents, technological adjuvants such as emulsifiers or plasticisers, active pharmacological ingredients, plant extracts, functional ingredients such as vitamins or mineral salts, and colorants.

The gum dough can be prepared according to any known methodology, for example with the use of continuous extruders or non-continuous mixing apparatus.

In one embodiment of the invention, the cooling flavour in the form of solid particulate matter and the cooling premix are inserted in the formulation of the chewing gum during preparation of the gum dough.

The cooling premix is a liquid at room temperature before preparation of the gum dough. During preparation of the gum dough, the premix is thoroughly mixed with, dispersed through, adsorbed onto the surface of and/or absorbed into the other ingredients, including the gum base and sweeteners. At the end of mixing, one or more regions constituted by the cooling premix are therefore no longer identifiable. During the mixing stage the interaction between the cooling premix and some ingredients can modify the properties of both. Without wishing to be constrained by theoretical factors, the Applicant considers that the cooling mixture comprising menthol esters and a lipophilic ingredient is able to interact in a particular way with the gum base, bonding to and partly penetrating into it, and is then released slowly during chewing, thus contributing to the cooling effect imparted by the gum of the invention.

If a discontinuous mixing equipment is employed, the ingredients of the gum dough, including the cooling flavour in the form of solid particulate matter and the cooling premix, can be added at the same time or at different times during manufacture. However, the cooling flavour in the form of solid particulate matter and the cooling premix are added at different times during the preparation of the gum dough.

If continuous extruders are used, the ingredients of the gum dough, including the cooling flavour in the form of solid particulate matter and the cooling premix, can be added in the same region or different regions of the extruder. However, the cooling flavour in the form of solid particulate matter and the cooling premix are added in different regions of the extruder. The transit time through the continuous extruder can be indicated as the time total (tₜₒₜ), and the ingredient addition times refer to the time that elapses between the feed zone of the first ingredient of the gum dough and the zone of insertion of the ingredient whose time is to be measured.

When the gum dough has been prepared, it is processed according to known methods to obtain a plurality of pieces of chewing gum or cores. The dough usually undergoes one or two consecutive additional extrusion stages.

Optionally, some ingredients of the gum dough, including the cooling flavour in the form of solid particulate matter and the cooling premix, can be added during the extrusion stages.

In order to form the plurality of chewing gum pieces or cores, after the additional extrusion stage or stages the mixture is conveyed to a subsequent rolling or moulding stage. Preferably, the cores obtained from the rolling or moulding stage present a discoidal, cylindrical, cubic or spherical geometrical configuration, with a longitudinal cross-section which is diamond-shaped, rectangular or rectangular with rounded corners (pillow-shaped).

Gum pieces are then processed according to known methods. Coated gums require the application of hard or soft coatings, as described below. In the case of uncoated gums (sticks and slabs), the subsequent stages mainly involve wrapping.

### Filling

The manufacturing process can also include a stage of introduction into the additionally extruded gum dough of a substance designed to fill the body of the chewing gum pieces, said introduction stage being performed after or at the same time as the additional extrusion stage, and before the rolling or moulding stage. The filling can be liquid, solid, granular or gelatinous. Depending on the type of core and filling material, and on the shape of the piece of chewing gum, the filling can be totally incorporated in the piece of chewing gum or can be visible from the exterior of the chewing gum on one or more sides.

In certain embodiments of the invention, the gum contains a quantity of filling completely surrounded by chewing gum. If the filling is liquid or granular, it exits rapidly from the gum during the first stages of chewing, thus contributing to the initial sensation imparted by the chewing gum. The presence in the liquid or granular filling of flavours, including menthol, and/or cooling agents, can increase the initial cooling sensation when chewing the chewing gum. Moreover, in the case of granular or powdered fillings, the use of ingredients with a negative heat of solution, such as dextrose, dextrose monohydrate, sorbitol, erythritol and xylitol, and mixtures thereof, contributes to the initial cooling sensation. The granular filling preferably comprises a particulate matter with at least one of the following: dextrose monohydrate, sorbitol, erythritol and xylitol, and mixtures thereof in crystals. The average particle size preferably ranges from 40 µm to 1000 µm, and even more preferably from 250 µm to 800 µm; moreover, the particulate matter may be present in quantities exceeding 10%, preferably 50%, and even more preferably exceeding 90% by weight of the filling.

The filling preferably also contains powdered flavours such as menthol and cooling agents, either "as is" or supported or encapsulated.

The filling in the form of particulate matter preferably comprises xylitol crystals with an average particle size ranging between 250 µm and 1000 µm, in a percentage exceeding 50 wt% of the filling.

Other embodiments require the filling not to be completely surrounded by chewing gum, but visible on one or more sides.

In one form of embodiment, the filling is visible on one side of the chewing gum only, occupying the space excavated on one side of the body of the chewing gum.

Alternatively, the filling can be visible on two opposite sides of a parallelepiped-shaped chewing gum. The filling can be square, parallelepipedal, round, elliptical, star-shaped, heart-shaped, etc.

In one form of embodiment the filling is visible on three sides of the chewing gum, occupying a groove excavated in the body of the chewing gum.

Alternatively, the filling can be visible on four sides of the chewing gum composition, in the form of a layer inserted between two layers of chewing gum. Even if there are more than three layers, the filling would be visible on four sides.

### Coating

### Hard and soft coating can be performed on the inventive gum centers, alone or in combination, according to known methods.

Flavours, intensive sweeteners, liquid or powdered colorants, optionally encapsulated or supported, either alone or mixed together, can be used in both types of coating.

Moreover, part of the coating can be obtained by one technique and part by another, thus generating infinite permutations of textures, colours, flavours, etc.

Both types of coating can be followed by a smoothing step and polishing step, which uses particular syrups and additives.

Particular coverings which can be used in conjunction with the present invention include coatings designed to cover particulates such as crystals of polyalcohols, by application of particular syrups, as described in US 8124145, or water, as described in WO 2009138208A1. Said coverings can also be applied after a hard or soft coating.

In a particular embodiment, the coating region is completed by applying a satin finishing to the outermost part.

The coating, completed in the traditional form or with a satin effect, can also contain glitter or speckles, well known in the art of chewing gum manufacture.

Other coatings use compounds based on fats, such as chocolate or imitations, or are obtained by immersion in molten compounds (such as molten xylitol). Said coatings can also be used within the scope of the invention, and combined with the preceding ones.

The chewing gum of the invention can also contain flavours in the coating region. In particular, the coating can contain a further cooling flavour comprising one or more of the following: menthol, menthol esters, p-menthane-carboxamides and mixtures thereof. A mixture containing the following substances, expressed as percentages of the finished product, is particularly preferred: monomenthyl glutarate 0.001% to 0.06%, dimenthyl glutarate 0.001% to 0.06%, and monomenthyl succinate 0.001% to 0.1 %. Optionally, carboxamides can be present in percentages ranging between 0.001 and 0.1% of the finished product.

The coating of the chewing gum can also contain a further cooling flavour in the form of particulate matter, comprising one or more of the following: menthol, menthol esters, p-menthane-carboxamides and mixtures thereof. In particular, the addition of powdered menthol, optionally containing anti-caking additives ranging between 0.01% and 1%, preferably between 0.07% and 0.3% (percentages of the finished product), is preferred.

### Examples 1-5

The following examples describe the composition of cooling flavours in the form of solid particulate matter usable in the invention. The percentages are percentages of the particulate matter.

| **Ingredient** | **%** | | | | |
|---|---|---|---|---|---|
| | **1** | **2** | **3** | **4** | **5** |
| Polyvinyl acetate MW 10000-20000 | 10 | | | 55 | 70 |
| Polyvinyl acetate MW 30000-50000 | | 50 | 60 | | |
| Polyvinyl acetate MW 60000-70000 | 30 | | | 5 | |
| Aspartame | 45 | | | | 19 |
| Neotame | | 10 | | 5 | |
| Alitame | | | 7 | | |
| N-ethyl-p-menthane carboxamide | | | | | 7 |
| N-ethoxycarbonylmethyl-p-menthane carboxamide | 10 | | | | |
| N-p-methoxyphenyl-p-menthane carboxamide | | | 13 | | |
| N-p-benzeneacetonitrile-menthane carboxamide | | 5 | | | |
| N-(2-(pyridin-2-yl)ethyl)-3-p-menthane carboxamide | | | | 8 | |
| Hydrogenated vegetable oil | | | 15 | | 3 |
| Mono- and diglycerides of fatty acids | | 10 | | | |
| Triethyl citrate | 5 | | | 13 | |
| Talc | | 24 | | 12 | |
| Silica | | | 5 | 2 | |
| E 133 | | 1 | | | |
| Vanillin | | | | | 1 |
| **Total** | 100 | 100 | 100 | 100 | 100 |

### Examples 6-8 - Cooling premix in liquid form

The following examples describe the composition of cooling premixes in liquid form usable in the invention. The percentages are percentages of the premix.

| **Ingredient** | **%** | | |
|---|---|---|---|
| | **6** | **7** | **8** |
| Monomenthyl glutarate | 25 | 12 | 24 |
| Dimenthyl glutarate | 10 | 5 | 6 |
| Monomenthyl succinate | 15 | 3 | 10 |
| Fractionated coconut oil | | 10 | |
| Triglycerides with chain C₂ | | 50 | 30 |
| Triglycerides with chain C₈-C₁₀ | | 20 | 30 |
| **Total** | 100 | 100 | 100 |

### Example 9-13 - Non-inventive and inventive chewing gums

The following examples report chewing gum formulations consisting of a region with gum base and sweeteners.

| **Ingredient** | **%** | | | | |
|---|---|---|---|---|---|
| | **9: non-inventive** | **10: non-inventive** | **11: non-inventive** | **12: non-inventive** | **13: inventive** |
| Gum base | 36.00 | 36.00 | 36.00 | 36.00 | 36.00 |
| Acesulfame K | 0.05 | 0.05 | 0.05 | 0.05 | 0.05 |
| Aspartame | 0.05 | 0.05 | 0.05 | 0.05 | 0.05 |
| Sucralose | 0.10 | 0.10 | 0.10 | 0.10 | 0.10 |
| **Cooling flavour in the form of solid particulate matter (Example 5)** | 0.00 | 0.00 | 0.00 | **2.00** | **2.00** |
| Mannitol | 10.00 | 10.00 | 10.00 | 10.00 | 10.00 |
| Glycerol | 3.50 | 3.00 | 3.00 | 3.00 | 3.00 |
| Peppermint flavour in solid form | 1.80 | 1.80 | 1.80 | 1.80 | 1.80 |
| Maltitol | 32.00 | 32.90 | 32.00 | 30.00 | 30.00 |
| **Cooling mixture consisting of menthol esters and lipophilic ingredients** (**Example 8)** | 0.00 | 0.00 | **1.50** | 0.00 | **1.50** |
| **Cooling mixture consisting of menthol esters** | 0.00 | **0.6** | 0.00 | 0.00 | 0.00 |
| Lipophilic ingredients of the cooling mixture | 0.00 | 0.00 | 0.00 | 1.50 | 0.00 |
| Maltitol syrup | 5.00 | 5.00 | 5.00 | 5.00 | 5.00 |
| Peppermint flavour | 1.50 | 0.50 | 0.50 | 0.50 | 0.50 |
| Sorbitol | 10.00 | 10.00 | 10.00 | 10.00 | 10.00 |
| **Total** | 100 | 100 | 100 | 100 | 100 |

The following examples report the percentage compositions of the inventive gum with filling regions, a region with gum base and at least one sweetener, and coating regions.

### Examples 14-16 - Fillings

| **Ingredient** | **%** | | |
|---|---|---|---|
| | **14** | **15** | **16** |
| Xylitol crystals, average particle size 500 µm | 87.00 | | 75.00 |
| Sorbitol crystals, average particle size 200 µm | 10.45 | | |
| Powdered menthol | 2.00 | | |
| Triacetin | 0.50 | | |
| E133 - aluminium lake | 0.05 | | 0.10 |
| Maltitol syrup | | 80.00 | 20.00 |
| Carboxymethylcellulose | | 0.20 | 1.00 |
| Sucralose | | 0.20 | |
| Acesulfame K | | 0.10 | |
| Glycerin | | 10.00 | |
| Water | | 8.00 | |
| Liquorice flavour (contains WS3) | | 1.50 | |
| Spearmint flavour | | | 1.50 |
| Water | | | 2.40 |
| **Total** | 100 | 100 | 100 |
| **Physical form** | Particulate matter | Liquid | Soft caramel |

### Examples 17-19 - Cores (region containing gum base and a sweetener)

| **Ingredient** | **%** | | |
|---|---|---|---|
| | **17 invention** | **18 invention** | **19 invention** |
| Gum base | 33.00 | 42.00 | 36.00 |
| Acesulfame K | 0.05 | 0.01 | 0.07 |
| Aspartame | 0.05 | 0.19 | 0.20 |
| Sucralose | 0.10 | | 0.03 |
| Xylitol | | 8.00 | 3.00 |
| Erythritol | | 15.00 | |
| Sorbitol | 8.00 | 22.00 | 15.00 |
| Mannitol | 15.00 | | 15.00 |
| Glycerol | 2.00 | 2.00 | |
| Maltitol | 30.00 | | 20.00 |
| Maltitol syrup | 6.00 | 1.00 | 3.00 |
| Mint flavour in solid form | 1.00 | | 0.50 |
| Mint flavour in liquid form | 0.50 | 2.00 | |
| Powdered menthol | | 1.50 | 1.00 |
| Cooling flavour in the form of solid particulate matter. example 1 | 3.50 | | |
| Cooling flavour in the form of solid particulate matter. example 2 | | 2.30 | |
| Cooling flavour in the form of solid particulate matter. example 5 | | | 1.00 |
| Cooling mixture. example 6 | 0.30 | | |
| Cooling mixture. example 7 | | 2.00 | |
| Cooling mixture. example 8 | | | 4.00 |
| Spray-dried N-p-benzeneacetonitrile-menthane carboxamide | 0.5 | | |
| N-(2-(pyridin-2-yl)ethyl)-3-p-menthane carboxamide and menthyl lactate | | | 0.20 |
| Powdered liquorice extract | | | 1.00 |
| Granules containing sage extract | | 2.00 | |
| **Total** | 100 | 100 | 100 |

### Examples 20-22 - Coating

| **Ingredient** | **%** | | |
|---|---|---|---|
| | **20** | **21** | **22** |
| Maltitol | 78.00 | | |
| Xylitol | | 60.00 | |
| Mannitol | | 15.50 | |
| Xylitol crystals. mean particle size 300 µm | | | 98.50 |
| Gum arabic | 16.00 | | |
| Gelatin | 2.30 | | |
| Mixture of starches and maltodextrins | | 6.00 | |
| Carboxymethylcellulose | | 3.00 | |
| Titanium dioxide | 1.50 | 2.50 | |
| Liquorice flavour | 0.60 | | |
| Peppermint flavour | | 2.00 | 1.30 |
| Spray-dried menthyl lactate | 0.20 | | |
| 10% menthyl succinate in propylene glycol | | 0.15 | |
| 10% N-ethoxycarbonylmethyl-p-menthane carboxamide in propylene glycol | 0.20 | | |
| Spray-dried N-p-benzeneacetonitrile-menthane carboxamide | | 0.05 | |
| Powdered menthol | | | 0.10 |
| Aspartame | 0.50 | 0.20 | 0.10 |
| Acesulfame K | 0.2 | 0.40 | |
| Brown speckles | 0.30 | | |
| Carnauba wax | 0.20 | 0.20 | |
| Mannitol/powdered gum arabic | | 10.00 | |
| **Total** | 100 | 100 | 100 |

### Examples 23-25 - Chewing gum with a plurality of regions

| **Ingredient** | **%** | | |
|---|---|---|---|
| | **23 invention** | **24 invention** | **25 invention** |
| Filling region | 8 (example 15) | 12 (example 14) | 30 (example 16) |
| Core | 60 (example 19) | 57 (example 17) | 65 (example 18) |
| Coating | 32 (example 20) | 30 (example 21) | 5 (example 22) |
| **Total** | 100 | 100 | 100 |
| **Type of chewing gum** | Liquid-filled dragee, with hard coating and glossy finishing. | Dragee with granular filling, hard coating and satin finishing. | Stick with layer of filling between two layers of gum. Granular coating as disclosed by WO2009/130820 8. |

### Example 26 - Organoleptic tests

Chewing gums 10, 11 and 12 (non-inventive), 13 and 24 (according to the invention) were subjected to organoleptic evaluation. Five expert tasters chewed the four gums for an hour, without being informed of the identity of the samples. At pre-set intervals (30 sec, 2, 5, 10, 20, 30, 40 and 50 min) they were asked to evaluate the cooling effect delivered by the product on the VAS scale (Visual Analogue Scale), with the values "no cooling" and "unbearable cooling" anchored to the ends of the scale. The scale contained 100 divisions, so that the cooling score could be measured on a scale of 0 to 100.

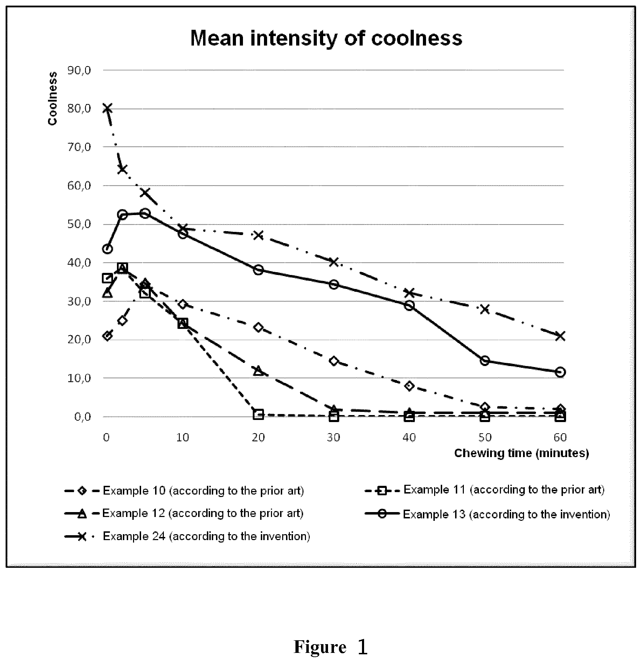

The mean results of the evaluation are set out in the graph in the Figure.

The non-inventive compositions, containing as cooling agents a mixture of menthol esters only (example 10), a cooling mixture with menthol esters and lipophilic ingredients only (example 11), or a cooling flavour in the form of solid particulate matter only (example 12), are generally less cool than the compositions of the invention (Examples 13 and 24). In particular, the non-inventive compositions have a cooling score of less than 20 from 30 minutes onwards, whereas inventive composition 13 has a cooling score of less than 20 from 50 minutes onwards, and inventive composition 24, which also contains some preferred embodiment such as additional cooling agents and filling and coating regions, has a cooling score higher than 20 for 60 minutes.

The chewing gum described in example 13 contains the cooling flavour in the form of solid particulate matter in the same quantity as Example 12, and the cooling mixture in the same quantity as Example 11. The other ingredients are substantially equal, so the cooling scores of example 13 would be expected to be equal to the sum of the cooling scores perceived for examples 11 and 12. The table, which shows the average perceived cooling scores, demonstrates that from 20 to 60 minutes' chewing, the cooling effect of example 13 is greater than the sum of the examples containing the individual ingredients of the invention, thereby demonstrating a synergistic effect on the perception of cold, due to the simultaneous presence in the formulation containing gum base and sweetener of a cooling flavour in the form of solid particulate matter and the cooling mixture.

**Table**

| Minutes of chewing | Cooling scores | | | | Synergi stic effect |
|---|---|---|---|---|---|
| | Example 11 (non-inventive) | Example 12 (non-inventive) | Total cooling Examples 11+12 | Example 13 (inventive) | |
| 0.5 | 36.0 | 32.3 | 68.3 | 43.6 | |
| 2 | 38.8 | 38.5 | 77.3 | 52.4 | |
| 5 | 32.3 | 34.8 | 67.0 | 52.8 | |
| 10 | 24.3 | 24.3 | 48.5 | 47.6 | |
| 20 | 0.5 | 12.0 | **12.5** | **38.2** | **yes** |
| 30 | 0.0 | 1.8 | **1.8** | **34.4** | **yes** |
| 40 | 0.0 | 1.0 | **1.0** | **29.0** | **yes** |
| 50 | 0.0 | 1.0 | **1.0** | **14.6** | **yes** |
| 60 | 0.0 | 1.0 | **1.0** | **11.6** | **yes** |

## Claims

1. Chewing gum imparting a cooling sensation to a consumer, comprising a region **characterised by** the presence of gum base, at least one sweetener, and a synergistic cooling combination of two separated components:
a) a solid particulate cooling flavour comprising a vinyl polymer, a dipeptide sweetener and at least one N-substituted p-menthane-carboxamide having formula I 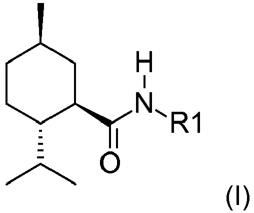 wherein R1 is selected from C1-C10 alkyl, C1-C10 alkoxycarbonylmethyl, heteroaryl-C1-C5 alkyl and phenyl, optionally substituted by C1-C5 alkoxy and/or cyano groups;
b) a cooling mix comprising one or more organic C3-C10 mono- and di- acids esterified with one or two menthol groups, homogeneously dispersed in said region.

2. Chewing gum according to claim 1, wherein said N-substituted p-menthane carboxamide is selected from N-ethyl-p-menthane carboxamide (WS3), N-ethoxycarbonylmethyl-p-menthane carboxamide (WS5), N-p-methoxyphenyl-p-menthane carboxamide (WS12), N-p-benzeneacetonitrile-menthanecarboxamide, N-(2-(pyridin-2-yl)ethyl)-3-p-menthanecarboxamide and combinations thereof.

3. Chewing gum according to claims 1-2, wherein said solid particulate cooling flavour has an N-substituted p-menthane-carboxamide : dipeptide sweetener : polyvinyl acetate ratio ranging from a minimum of 0.02:1:0.75 to a maximum of 8:1:9.

4. Chewing gum according to claims 1-2, wherein said solid particulate cooling flavour comprises 1% to 10% of said N-substituted p-menthane-carboxamide, 5% to 50% of said dipeptide sweetener and 30% to 90% of said vinyl polymer, expressed as a percentage of said particulate.

5. Chewing gum according to claims 1-4, wherein said chewing gum comprises 0.1 % to 10% of said solid particulate cooling flavour, preferably 1% to 5% thereof, expressed as a percentage of said chewing gum.

6. Chewing gum according to claim 1 wherein said cooling mix contains, as organic acids esterified with one or more menthol groups, one or more of the following: monomenthyl glutarate, dimenthyl glutarate, monomenthyl succinate and mixtures thereof.

7. Chewing gum according to claim 6, wherein said cooling mix comprises 1% to 70% monomenthyl glutarate, 1% to 70% dimenthyl glutarate and 0.5% to 50% monomenthyl succinate, expressed as a percentage of said mix.

8. Chewing gum according to claims 6 and 7, wherein said cooling mix also contains a lipophilic ingredient.

9. Chewing gum according to claims 1, 6, 7 and 8, containing 0.2% to 5% of said liquid cooling mix.

10. Chewing gum according to claims 1-9, further comprising a filling region, a coating region and combination thereof.

11. Chewing gum according to claims 1-10, comprising an additional cooling flavour in spray-dried, liquid or encapsulated form, wherein the flavouring substances comprise at least one of menthane carboxamides and menthol esters.

12. Chewing gum according to claims 10 and 11, comprising within the coating region an additional cooling flavour comprising one or more of menthol, menthol esters, p-menthane carboxamides and mixtures thereof.

13. Chewing gum according to claims 10 and 12, comprising within the coating region an additional cooling flavour in particulate form comprising one or more of menthol, menthol esters, p-menthane carboxamides and mixtures thereof.

14. Chewing gum according to claims 1-13, wherein said cooling flavour in solid particulate form is contained in said chewing gum in a percentage from 0.1 % to 10%, preferably from 1% to 5%, and wherein said cooling mix constitutes 0.2% to 5% of the chewing gum.

15. Chewing gum according to claims 1 and 4, wherein said vinyl polymer is selected from polyvinyl acetate, polyvinyl acetate/polyvinyl laurate copolymer, polyvinyl acetate/polyvinyl versatate/polyvinyl alcohol terpolymer, polyvinylpyrrolidone, crosslinked polyvinylpyrrolidone, polyvinyl alcohol and mixtures thereof.

16. Process for the manufacture of the chewing gum according to claims 1-15, comprising the manufacture of a dough through the addition in a mixer, and a mixing operation carried out for a time tₜₒₜ, of the following ingredients:
I. gum base;
II. at least one sweetener;
III. a solid particulate cooling flavour comprising a vinyl polymer, a dipeptide sweetener and at least one N-substituted p-menthane-carboxamide having formula I 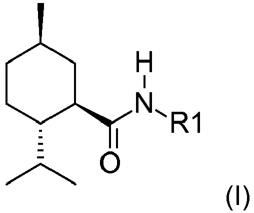 wherein R1 is selected from C1-C10 alkyl, C1-C10 alkoxycarbonylmethyl, heteroaryl-C1-C5 alkyl or phenyl, optionally substituted by C1-C5 alkoxy and/or cyano groups;
IV. a liquid cooling premix comprising one or more organic C3-C10 mono- and di-acids esterified with one or two menthol groups, and optionally at least one lipophilic component, evenly dispersed in said region;
wherein ingredients III) and IV) are added at two distinct times t_{III}<tₜₒₜ and t_{IV}<tₜₒₜ.

17. Process according to claim 16, wherein the absolute difference between t_{III} and t_{IV} is higher than 30 seconds, preferably higher than 60 seconds.

18. Process according to claims 16 or 17, further comprising reducing the dough to a plurality of chewing gum pieces or centres through one or more of the extrusion, die forming, cutting, rolling and scoring.

## Patentansprüche

1. Kaugummi, das einem Konsumenten ein Kühlgefühl übermittelt, umfassend einen Bereich der charakterisiert ist durch die Anwesenheit einer Gummigrundlage, mindestens einem Süßungsmittel und einer synergistischen Kühlkombination aus zwei separaten Komponenten:
a) Einem festen partikelförmigen kühlenden Aroma, umfassend ein Vinylpolymer, ein Dipeptid-Süßungsmittel und mindestens ein N-substituiertes p-Menthancarboxamid der Formel (I) 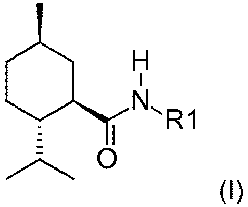 worin R1 ausgewählt wurde aus der Gruppe bestehend aus C1-C10 Alkyl, C1-C10 Alkoxycarbonylmethyl, heteroaryl-C1-C5 Alkyl und Phenyl, optional substituiert mit C1-C5 Alkoxy- und/oder Cyanogruppen;
b) eine Kühlmischung umfassend eine oder mehrere organische C3-C10 mono- und di-Säuren, verestert mit einer oder zwei Mentholgruppen, homogen verteilt im besagtem Bereich.

2. Kaugummi nach Anspruch 1, wobei besagtes N-substituiertes p-Menthancarboxamid ausgewählt wurde aus der Gruppe bestehend aus N-Ethyl-p-Menthancarboxamid (WS3), N-Ethoxycarbonylmethyl-p-Menthancarboxamid (WS5), N-p-Methoxyphenyl-p-Menthancarboxamid (WS12), N-p-Benzenacetonitril-Menthancarboxamid, N-(2-(Pyridin-2-yl)ethyl)-3-p-Menthancarboxamid und Kombinationen hiervon.

3. Kaugummi nach einem der Ansprüche 1 oder 2, wobei das feste partikelförmige kühlende Aroma ein Verhältnis von N-substituiertem p-Menthan-Carboxamid : Dipeptid-Süßungsmittel : Polyvinylacetat in einem Bereich von mindestens 0,02:1: 0,75 bis maximal 8:1:9 aufweist.

4. Kaugummi nach einem der Ansprüche 1 oder 2, wobei das genannte feste partikelförmige kühlende Aroma 1% bis 10% des genannten N-substituierten p-Menthancarboxamid, 5% bis 50% des genannten Dipeptid-Süßungsmittel und 30% bis 90% des genannten Vinyl Polymers umfasst, wobei die Werte als prozentualer Anteil des genannten Partikels ausgedrückt sind.

5. Kaugummi nach einem der Ansprüche 1-4, wobei das genannte Kaugummi 0,1% bis 10% des genannten festen partikelförmigen kühlenden Armoas, vorzugsweise 1% bis 5% hiervon umfasst, wobei die Werte als prozentualer Anteil des Kaugummis ausgedrückt sind.

6. Kaugummi nach Anspruch 1, wobei die genannte Kühlmischung als, die mit einer oder mehreren Mentholgruppen veresterte organische Säure, eine oder mehrere der folgenden enthält: Monomenthylglutarat, Dimenthylglutarat, Monomenthylsuccinat und Mischungen hiervon.

7. Kaugummi nach Anspruch 6, wobei die genannte Kühlmischung 1% bis 70% Monomenthylglutarat, 1% bis 70% Dimenthylglutarat und 0,5 bis 50% Monomenthylsuccinat umfasst, wobei die Werte als prozentualer Anteil der genannten Mischung ausgedrückt sind.

8. Kaugummi nach Anspruch 6 und 7, wobei die genannte Kühlmischung auch einen lipophilen Bestandteil enthält.

9. Kaugummi nach den Ansprüchen 1, 6, 7 und 8 enthaltend 0,2% bis 5% der genannten flüssigen Kühlmischung.

10. Kaugummi nach einem der Ansprüche 1-9, weiterhin umfassend eine Füllregion, eine Beschichtungsregion und Kombinationen hiervon.

11. Kaugummi nach einem der Ansprüche 1-10, umfassend ein zusätzliches kühlendes Aroma in sprühgetrockneter, flüssiger oder verkapselter Form, wobei die Aromastoffe mindestens einen aus der Gruppe von Menthancarboxamiden und Mentholestern umfassen.

12. Kaugummi nach Anspruch 10 und 11, umfassend innerhalb der Beschichtungsregion ein zusätzliches kühlendes Aroma, umfassend eines oder mehreren von Menthol, Mentholester, p-Menthancarboxamide und Mischungen hiervon.

13. Kaugummi nach Anspruch 10 und 12 umfassend innerhalb der Beschichtungsregion ein zusätzliches kühlendes Aroma in Partikelform umfassend eine oder mehrere aus Menthol, Mentholester, p-Menthancarboxamide und Mischungen hiervon.

14. Kaugummi nach Anspruch 1-13, wobei das genannte feste partikelförmige kühlende Aroma im genannten Kaugummi in einem Prozentanteil von 0,1% bis 10%, vorzugsweise von 1% bis 5% enthalten ist und wobei die genannte Kühlmischung 0,2% bis 5% des Kaugummis ausmacht.

15. Kaugummi nach einem der Ansprüche 1 und 4, wobei das genannte Vinylpolymer ausgewählt wurde aus der Gruppe bestehend aus Polyvinylacetat, Polyvinylacetat / Polyvinyllaurat Copoylmer, Polyvinylacetat / Polyvinylversatat / Polyvinylalkohol Terpolymer, Polyvinylpyrrolidon, vernetztes Polyvinylpyrrolidon, Polyvinylalkohol und Mischungen hiervon.

16. Verfahren zur Herstellung eines Kaugummis nach einem der Ansprüche 1-15, umfassend die Herstellung eines Teigs durch die Zugabe in einen Mischer und einem Mischvorgang, durchgeführt für eine Zeit tₜₒₜ für die folgenden Inhaltsstoffe:
I. Gummigrundlage
II. Mindestens ein Süßungsmittel
III. Ein festes partikelförmiges kühlendes Aroma umfassend ein Vinylpolymer, ein Dipeptid-Süßungsmittel und mindestens ein N-substituiertes p-Menthancarboxamid der Formel (I) 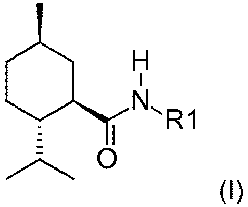 worin R1 ausgewählt wurde aus der Gruppe bestehend aus C1-C10 Alkyl, C1-C10 Alkoxycarbonylmethyl, heteroaryl-C1-C5 Alkyl oder Phenyl, optional substituiert mit C1-C5 Alkoxy- und/oder Cyanogruppen;
IV. Eine flüssige Kühlvormischung umfassend eine oder mehrere organische C3-C10 mono- und di-Säuren, die mit einer oder zwei Mentholgruppen verestert sind und optional mindestens einen lipophilen Bestandteil, der gleichmäßig im genannten Bereich verteilt ist;
wobei die Inhaltsstoffe III) und IV) an zwei unterschiedlichen Zeitpunkten t_{III}<tₜₒₜ und t_{IV}< tₜₒₜ hinzugegeben werden.

17. Verfahren nach Anspruch 16, wobei die absolute Differenz zwischen t_{III} und t_{IV} größer als 30 Sekunden, vorzugsweise größer als 60 Sekunden ist.

18. Verfahren nach einem der Ansprüche 16 oder 17, weiterhin umfassend die Zerkleinerung des Teigs zu einer Vielzahl an Kaugummistücken oder Zentren durch eine oder mehrere der Extrusion, Gesenkformen, Walzen und Ritzen.

## Revendications

1. Gomme à mâcher conférant une sensation de fraîcheur à un consommateur, comprenant une région **caractérisée par** la présence d'une base de gomme, d'au moins un édulcorant, et d'une combinaison rafraîchissante agissant en synergie de deux composants séparés :
a) une flaveur rafraîchissante sous forme de particule solide comprenant un polymère de vinyle, un édulcorant dipeptidique et au moins un *p*-menthane-carboxamide N substitué de formule I 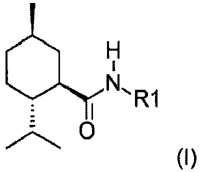 dans laquelle R1 est sélectionné parmi un groupe alkyle en C₁ à C₁₀, un groupe alcoxycarbonylméthyle en C₁ à C₁₀, un groupe hétéroaryle-(alkyle en C₁ à C₅) et un groupe phényle, éventuellement substitué par des groupes alcoxy en C₁ à C₅ et/ou des groupes cyano ;
b) un mélange rafraîchissant comprenant un ou plusieurs mono- et diacides organiques en C₃ à C₁₀ estérifiés avec un ou deux groupes menthol, dispersés de manière homogène dans ladite région.

2. Gomme à mâcher selon la revendication 1, dans laquelle ledit *p-*menthane carboxamide N substitué est sélectionné parmi le N-éthyl-*p-*menthane carboxamide (WS3), le N-éthoxycarbonylméthyl-*p*-menthane carboxamide (WS5), le N-*p*-méthoxyphényl-*p*-menthane carboxamide (WS12), le N-*p*-benzèneacétonitrile-menthane carboxamide, le N-(2-(pyridin-2-yl)éthyl)-3-*p*-menthanecarboxamide et leurs combinaisons.

3. Gomme à mâcher selon les revendications 1 à 2, dans laquelle ladite flaveur rafraîchissante sous forme de particule solide présente un rapport du *p-*menthane-carboxamide N substitué:édulcorant dipeptidique:poly(acétate de vinyle) se situant à partir d'un minimum de 0,02:1:0,75 à un maximum de 8:1:9.

4. Gomme à mâcher selon les revendications 1 à 2, dans laquelle ladite flaveur rafraîchissante sous forme de particule solide comprend de 1 % à 10 % dudit *p*-menthane-carboxamide N substitué, de 5 % à 50 % dudit édulcorant dipeptidique et de 30 % à 90 % dudit polymère de vinyle, exprimé en pourcentage de ladite particule.

5. Gomme à mâcher selon les revendications 1 à 4, dans laquelle ladite gomme à mâcher comprend de 0,1 % à 10 % de ladite flaveur rafraîchissante sous forme de particule solide, préférablement de 1 % à 5 % de celle-ci, exprimé en pourcentage de ladite gomme à mâcher.

6. Gomme à mâcher selon la revendication 1 dans laquelle ledit mélange rafraîchissant contient, comme acides organiques estérifiés avec un ou plusieurs groupes menthol, un ou plusieurs des suivants : glutarate monomenthylique, glutarate dimenthylique, succinate monomenthylique et leurs mélanges.

7. Gomme à mâcher selon la revendication 6, dans laquelle ledit mélange rafraîchissant comprend de 1 % à 70 % de glutarate monomenthylique, 1 % à 70 % de glutarate dimenthylique et de 0,5 % à 50 % de succinate monomenthylique, exprimé en pourcentage dudit mélange.

8. Gomme à mâcher selon les revendications 6 et 7, dans laquelle ledit mélange rafraîchissant contient également un ingrédient lipophile.

9. Gomme à mâcher selon les revendications 1, 6, 7 et 8, contenant de 0,2 % à 5 % dudit mélange rafraîchissant liquide.

10. Gomme à mâcher selon les revendications 1 à 9, comprenant en outre une région de garniture, une région d'enrobage et leur combinaison.

11. Gomme à mâcher selon les revendications 1 à 10, comprenant une flaveur rafraîchissante additionnelle sous la forme séchée par pulvérisation, liquide ou encapsulée, les substances aromatisantes comprenant au moins l'un parmi les carboxamides de menthane et les esters de menthol.

12. Gomme à mâcher selon les revendications 10 et 11, comprenant au sein de la région d'enrobage une flaveur rafraîchissante additionnelle comprenant un ou plusieurs parmi le menthol, les esters de menthol, les *p*-menthane carboxamides et leurs mélanges.

13. Gomme à mâcher selon les revendications 10 et 12, comprenant au sein de la région d'enrobage une flaveur rafraîchissante additionnelle sous forme de particule comprenant l'un ou plusieurs parmi le menthol, les esters de menthol, les *p*-menthane carboxamides et leurs mélanges.

14. Gomme à mâcher selon les revendications 1 à 13, dans laquelle ladite flaveur rafraîchissante sous forme de particule solide est contenue dans ladite gomme à mâcher sous un pourcentage de 0,1 % à 10 %, préférablement de 1 % à 5 %, et ledit mélange rafraîchissant constitue de 0,2 % à 5 % de la gomme à mâcher.

15. Gomme à mâcher selon les revendications 1 et 4, dans laquelle ledit polymère de vinyle est sélectionné parmi le poly(acétate de vinyle), le copolymère de poly(acétate de vinyle)/poly(laurate de vinyle), le terpolymère de poly(acétate de vinyle)/poly(versatate de vinyle)/poly(alcool de vinyle), la polyvinylpyrrolidone, la polyvinylpyrrolidone réticulée, le poly(alcool de vinyle) et leurs mélanges.

16. Procédé de fabrication de la gomme à mâcher selon les revendications 1 à 15, comprenant la fabrication d'une pâte par l'addition dans un mélangeur, et une opération de mélange conduite sur une durée tₜₒₜ des ingrédients suivants :
I. gomme de base;
II. au moins un édulcorant ;
III. une flaveur rafraîchissante sous forme de particule solide comprenant un polymère de vinyle, un édulcorant dipeptidique et au moins un *p*-menthane-carboxamide N substitué de formule I 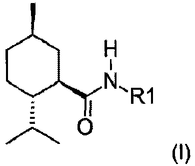 dans laquelle R1 est sélectionné parmi un groupe alkyle en C₁ à C₁₀, un groupe alcoxycarbonylméthyle en C₁ à C₁₀, un groupe hétéroaryle-(alkyle en C₁ à C₅) ou un groupe phényle, éventuellement substitué par des groupes alcoxy en C₁ à C₅ et/ou des groupes cyano ;
IV. un prémélange rafraîchissant liquide comprenant un ou plusieurs mono- et diacides organiques en C₃ à C₁₀ estérifiés avec un ou deux groupes menthol, et éventuellement au moins un composant lipophile, dispersé de manière uniforme dans ladite région ;
les ingrédients III) et IV) étant ajoutés à deux moments distincts t_{III} < tₜₒₜ et t_{IV} < tₜₒₜ.

17. Procédé selon la revendication 16, la différence absolue entre t_{III} et t_{IV} étant supérieure à 30 secondes, préférablement supérieure à 60 secondes.

18. Procédé selon les revendications 16 ou 17, comprenant en outre la réduction de la pâte en une pluralité de morceaux ou de centres de gomme à mâcher par l'intermédiaire de l'un ou plusieurs parmi l'extrusion, la mise en forme à la filière, la découpe, le roulage et l'incisage.
